# EUROPEAN PATENT SPECIFICATION

(11) **EP 0 749 968 B1**
(45) Date of publication and mention of the grant of the patent: **11.10.2000**
(21) Application number: 96108604.8
(22) Date of filing: 30.05.1996
(51) Int. Cl.: C07D 303/24, C08G 59/24

(54) **Epoxy resin and process for producing the same**
Epoxidharz und Verfahren zu ihrer Herstellung
Résine époxyde et procédé de sa préparation

(30) Priority: 02.06.1995 JP 13676095
(43) Date of publication of application: 27.12.1996
(73) Proprietor: SUMITOMO CHEMICAL COMPANY LIMITED, Osaka-shi, Osaka 541-0041 (JP)
(72) Inventor: Yokota, Akira, Tsukuba-shi, Ibaraki (JP); Hirano, Yasuhiro, Tsukuba-shi, Ibaraki (JP); Akiba, Masatsugu, Tsukuba-shi, Ibaraki (JP); Nakamura, Hiroshi, Tsuchiura-shi, Ibaraki (JP); Naitoh, Shigeki, Tsukuba-shi, Ibaraki (JP)
(74) Representative: Henkel, Feiler, Hänzel

(56) References cited:
- DE-A- 2 041 221
- ACS SYMP. SER.1979, vol. 114, 1979, pages 259-262, XP000603617 J.R. GRIFFITH: "Epoxy resins containing a specific vulnerability"
- MAKROMOL. CHEM., RAPID COMMUN., vol. 2, no. 5, 1981, pages 411-415, XP002015022 Y. OSADA, E.A.: "Photomechanochemical energy conversion in a polyamide containing a stilbene structure in the backbone "

## Description

### Field of the Invention

The present invention relates to an epoxy resin, which is useful for adhesives, paints, electric and electronic materials such as insulation materials and laminated plates, particularly useful to encapsulate the electronic devices, and a process for producing the same.

### Background of the Invention

There are many research papers on epoxy resins having a stilbene skeleton. For example, ACS symposium series 114, chap17, pp259-262(176th meeting of the American Chemical Society(1978)) discloses the epoxy resins having an unsubstituted stilbene skeleton. And glycidyl ether derivatives of bisphenol compounds having a stilbene skeleton, unsubstituted or substituted by alkyl group(s), and preferably symmetrically substituted by methyl groups, are disclosed (US patent No. 4762901, German patent No. 3622613, Japanese Patent,Kokai(Laid open) No. 63-23931)

The epoxy resins having a stilbene skeleton disclosed hitherto have no distinction between the *trans*-isomer and the *cis*-isomer, but the stilbene skeleton obtained by the ordinary process has 100% of *trans*-configuration.

These *trans*-stilbene type epoxy resins have problems that the ways of using are limited because of the low operation efficiency in processing them. For example, because of the high melting points of the *trans*-stilbene type resins in general, the working process like kneading etc. becomes very difficult. Concretely, since the glycidyl derivatives of 4, 4'-dihydroxystilbene, 4, 4'-dihydroxy-3, 3'-dimethylstilbene, and 4, 4'-dihydroxy-3, 3', 5, 5'-dimethylstilbene have high melting points of 208-215°C, 150°C,and 151°C respectively, there are problems with the compounds that it is difficult to mix homogeneously with epoxy curing agents and to knead homogeneously with the other components. Whereas the stilbene type epoxy resins have comparatively low viscosity under molten condition, and it is easy to knead with other components due to this property of low viscosity. Therefore, further lowering of the viscosity means further improvement of the efficiency of the kneading process.

### Summary of the Invention

The object of the present invention is the improvement of the high melting point of the stilbene type epoxy resin to lower, and the achievement of the lower viscosity than that of the *trans*-isomer.

In view of the above situation, the present inventors had studied on the means to control the properties of the stilbene type epoxy resins, for example, to lower the melting point and viscosity. As a result, the present inventors found out that the stilbene type epoxy resin containing more than a certain amount of the *cis*-isomer, which was the other isomer of the *trans*-isomer obtained hitherto, met the object, and accomplished the present invention.

The present invention provides a epoxy resin and a process for producing it as described below.

The present invention is as follows:
[1] A epoxy resin represented by the general formula (1) consisting of 100 mol% of the *cis*-isomer, or 10 mol% or more of the *cis*-isomer and the rest of the *trans*-isomer 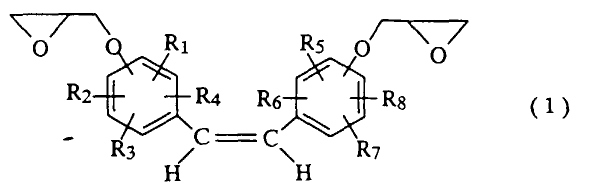 wherein R₁ to R₈ are independently an acyclic or cyclic alkyl group having 1 to 6 carbon atoms,hydrogen atom or a halogen atom.
[2] A process for producing a epoxy resin described above [1], characterized by the irradiation of a *trans*-stilbene type epoxy resin represented by the general formula (2) with ultra-violet light 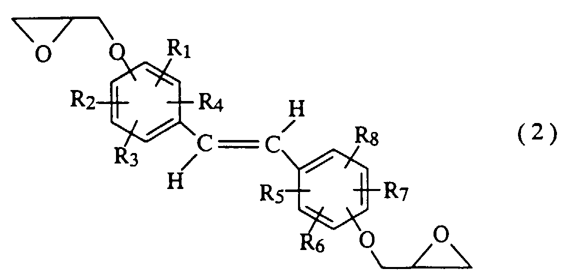 wherein R₁ to R₈ are independently an acyclic or cyclic alkyl group having 1 to 6 carbon atoms, hydrogen atom or a halogen atom.
[3] A process for producing an epoxy resin described above [1], characterized by the irradiation of a *trans*-stilbene type phenol represented by the general formula (3) 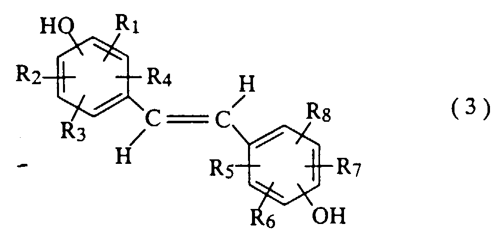 wherein R₁ to R₈ are independently an acyclic or cyclic alkyl group having 1 to 6 carbon atoms, hydrogen atom or a halogen atom, with ultra-violet light to obtain a stilbene type phenol represented by the general formula (4) consisting of 100 mol% of the *cis*-isomer, or 10 mol% or more of the *cis*-isomer and the rest of the *trans*-isomer 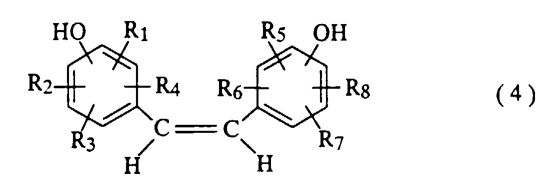 wherein R₁ to R₈ are as defined in the general formula (3), and by the reaction of the phenol of the general formula (4) with epihalohydrin in the presence of basic substance.

### Brief Description of the Figures

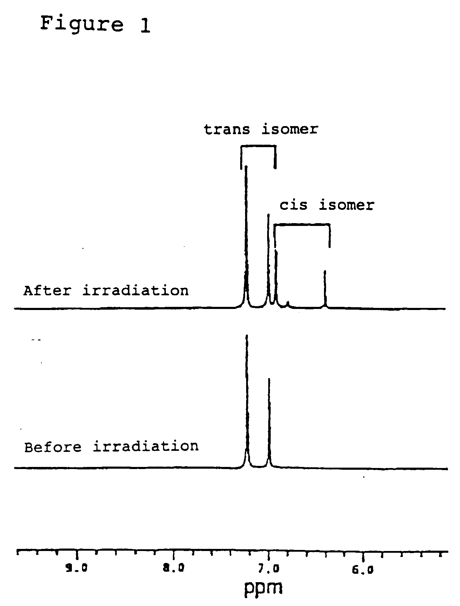

Fig 1 shows ¹H-NMR spectrum indicating the reaction ratio, before and after irradiation in the photo isomerization reaction in Example 1.

### Detailed Description of the Invention

The substituents, R₁-R₈ of the stilbene type epoxy resin represented by the general formula (1) or (2), in the present invention, are exemplified concretely by hydrogen atom, a methyl group, an ethyl group, a propyl group, a butyl group, an amyl group, a hexyl group, a cyclohexyl group (including each isomer), a chlorine atom and a bromine atom, etc. Preferably the substituents are hydrogen, a methyl group, an ethyl group, a propyl group and a butyl group because of the low melt viscosity of the products and the availability of law materials.
The substituents of the stilbene type phenol represented by the general formula (3) or (4) are the same as those described above.

The stilbene type epoxy resin represented by the general formula (1), consisting of 100 mol% of the *cis*-isomer, or 10 mol% or more of the *cis*-isomer and the rest of the *trans*-isomer, can be produced by the irradiation with ultra-violet light and the resulting photo isomerization reaction of the *trans*-stilbene type epoxy resin represented by the general formula (2). The *trans*-stilbene type epoxy resin represented by the general formula (2), is obtained by the well-known process of the glycidyl etherification of a phenol derivative. In other words, the epoxy resin is obtained by the reaction of the *trans*-stilbene type phenol with epihalohydrin in the presence of an alkali such as sodium hydroxide etc. Particularly, in order to obtain the highly pure product, the reaction is preferably conducted in an aprotic solvent or a solvent like dioxane etc. as disclosed in Japanese patent kokai(laid open) No.60-31517 .

The stilbene type phenol represented by the general formula (3) in the present invention, can be produced by the rearrangement reaction of a chloro derivative prepared by the reaction of monochloroacetaldehyde and phenol (Liebigs Ann. Chem. 730, 31(1969)), the rearrangement reaction of a chloro derivative prepared by the reaction of chloral and phenol ((Ann., 325, 26(1902)), the reduction of a stilbenequinone (J. Org. Chem., 18, 261(1953)), the process using quinonemethide as a starting material(J. Org. Chem., 34(11), 3404(1969)), the de-methylation of dimethoxystilbene (Recl. Trav. Chim. Pays-Bas., 80, 775(1961)), or the condensation reaction of a benzaldehyde derivative (Synthesis, 1989, 883 etc.) etc.

The *trans*-stilbene type phenol is represented by the general formula(3). Examples thereof include the *trans*-isomers of 4, 4'-dihydroxystilbene, 4, 4'-dihydroxy-3,3'-dimethylstilb ene, 3, 3'-diethyl-4, 4'-dihydroxystilbene, 4, 4'-dihydroxy-3,3'-dipropylstilbene, 3, 3d'-diamyl-4, 4'-dihydroxystilbene,3,3'-dihexyl-4, 4'-dihydroxystilbene, 3, 3'-dicyclohexyl-4,4'-dihydroxystilbene, 2, 2'-dihydroxy-3, 3', 5, 5'-tetramethylstilbene, 4, 4'-dihydroxy-3, 3', 5, 5'-tetramethylstilbene, 4,4'-dihydroxy-3,3'-di-t-butylstilbene , 4, 4'-dihydroxy-3,3'-di-t-butyl-5, 5'-dimethylstilbene, 4, 4'-dihydroxy-3,3'-di-t-butyl-6, 6'-dimethylstilbene, 2, 2'-dihydroxy-3, 3'-di-t-butyl-6, 6'-dimethylstilbene, 2, 4'-dihydroxy-3, 3'-di-t-butyl-6,6'-dimethylstilbene, and 4, 4'-dihydroxy-3, 3', 5,5'-tetra-t-butylstilbene with these *trans*-isomer (including the isomers of the substituted positions). Particurarly favoured are the *trans*-isomers of, 4, 4'-dihydroxystilbene, 2, 2'-dihydroxy-3, 3', 5, 5'-tetramethylstilbene, 4, 4'-dihydroxy-3, 3', 5, 5'-tetramethylstilbene, 4, 4'-dihydroxy-3, 3'-di-t-butyl-5, 5'-dimethylstilbene, 4, 4'-dihydroxy-3, 3'-di-t-butyl-6, 6'-dimethylstilbene, 2, 2'-dihydroxy-3, 3'-di-t-butyl-6, 6'-dimethylstilbene, and 2, 4'-dihydroxy-3, 3'-di-t-butyl-6, 6'-dimethylstilbene etc., from the viewpoints of the easiness of the synthesis, properties, and the cost of starting materials.

The photoisomerization reaction of the *trans*-stilbene type epoxy resin proceeds by the irradiation of ultra-violet light with a UV emission equipment under solution, melt or solid condition.

Examples of the solvent for the photoisomerization include hydrocarbons such as toluene and xylene etc., halogenated hydrocarbons such as chlorobenzene etc., ethers such as dioxane, tetrahydrofuran etc., alcohols such as methanol, ethanol, propanol etc., ketones such as methyl isobutyl ketone, methyl ethyl ketone etc., aprotic polar solvents such as dimethylsulfoxide, dimethyacetamide, dimethyformamide etc., glycols such as ethylene glycol, propylene glycol etc., acidic solvents such as acetic acid etc.,and the like. The above solvents can be used alone or in combination.

Examples of the irradiation equipment for the photoisomerization reaction include a low-pressure mercury lamp, a high-pressure mercury lamp, a Xenon lamp and the lasers such as excimer laser. As the efficiency of the isomerization reaction is influenced by the absorbance of the absorption band of the compound and the wave length of emission lines of the light source, it is preferable to select the light source by matching to the wavelength range of the absorption band in ultra-violet and visible regions of the *trans*-isomer. Concretely the region of the wavelength of the emission lines to be utilized is 250 to 400nm.

The stilbene type epoxy resin consisting of 10 mol% or more of the *cis*-isomer in the present invention, can be produced by the photoisomerization reaction resulting from the UV light irradiation of the *trans*-stilbene type phenol represented by the general formula (3) to obtain the stilbene type phenol consisting of 10 mol% or more of the *cis*-isomer represented the formula (4), and by the reaction of this stilbene type phenol with epihalohydrin in the presence of basic substance. The reaction can be conducted similarly as the photoisomerization of the *trans*-stilbene type phenol.

And the glycidyl etherification reaction of the stilbene type phenol consisting of 10 mol% or more of the *cis*-isomer, can be conducted according to the well known process of the glycidyl etherification of the phenols as described above.

One of the most convenient methods of the identification of the *cis*-stilbene type epoxy resins utilizes ¹H-NMR spectra. Thus, in the region of the protons attached to an unsaturated bond on the ¹H NMR spectra, both peaks assigned to the protons attached to a C=C bond and an aromatic ring of the cis-isomer shift to a higher field than those of the *trans* isomer. Especially, the peaks of the *cis*-isomer assigned to the proton attached to a C=C bond are observed at the high field region of 6.5 ppm or less, where the peaks of the *trans*-isomer are not observed.

In the present invention, the isomerization ratio from the *trans*-isomer to the *cis*-isomer is defined by (The number of mole of the *cis*-isomer / ( The number of mole of the *cis*-isomer + The number of mole of the *trans* isomer )) x 100. In order to obtain the objective properties in the present invention, the isomerization ratio is 10 mol% or more, preferably 20 mol% or more, and more preferably 40 mol% or more. The isomerization ratio of 10 mol% or less is too low to obtain the objective properties due to the lack of the content of the *cis*-isomer molecules.

To obtain a pure cis-isomer, purification such as recrystallization, fractional crystallization or chromatography is necessary.

### Examples

The examples of the present invention are exhibited below. However, the present invention is not restricted to these examples.

### Example 1

A methyl isobutyl ketone solution containing 5% of epoxy resin of 4, 4'-dihydroxy-3, 3', 5, 5'-tetramethylstilbene( epoxy resin consisting fully of *trans*-isomer), which was obtained by the ordinary method starting from 2, 6-xylenol and chloroacetaldehyde, was irradiated with ultra-violet light by a high-pressure mercury lamp for the photoisomerization. After the isomerization, the solution was distilled under reduced pressure to afford a yellow material. The ¹H-NMR spectra of the reactant before and after the irradiation in this experiment were illustrated in Figure 1. The NMR spectra were measured by Brucker AC200P spectrometer. From these spectra, the appearance of the peaks at high field characteristic to the *cis*-olefin but never observed before irradiation was confirmed after the irradiation. Namely, in the ¹H-NMR spectrum, the *cis*-stilbene type epoxy is characterized by the appearance of the peaks at high field of 7 ppm or less due to the proton attached to an unsaturated bond. Furthermore, from the intensity of the peaks in the spectrum, the ratio of the isomerization to the *cis*-isomer can be calcurated. In this sample, it was determined that 27% was converted to the *cis*-isomer. As a result of the irradiation of the 0.1% solution of the same sample, the objective material containing 50% of the isomelized *cis*-isomer was obtained. Table 1 shows the melting points of these resins obtained together with that of the resin before irradiation. The melting points were measured by Yanagimoto MP-S3 melting point measurement apparatus, and the melting point was determined to be the temperature at which the sample completely melted.

**[Table 1]**

| The relationship between the *cis* isomerization ratio and the melting point. | |
|---|---|
| *cis* isomerization ratio (%) | melting point (°C) |
| 0 | 151 |
| 27 | 135 |
| 50 | 120 |

Table 2 shows the temperature dependence of the viscosity of the resin having the *cis* isomerization ratio of 27% and the one before irradiation. The viscosity was measured by Contraves Leomat 115A viscosity measurement apparatus.

**[Table 2]**

| The temperature dependence of the viscosity before and after photo irradiation | | | |
|---|---|---|---|
| *cis* isomerization ratio (%) | viscosity (poise) | | |
| | 150°C | 160°C | 170°C |
| 0 | - | 0.17 | 0.10 |
| 27 | 0.15 | 0.08 | 0.04 |

The stilbene type epoxy resin of the present invention, consisting of 10 mol% or more of the *cis*-isomer, shows different properties from those of the whole *trans* stilbene type epoxy resin in respects of lower melting point and viscosity. In the photo-isomerization reaction, it becomes possible to control the properties by the change of the photo irradiation condition. By the control of the properties of the resin as described above, the operation efficiency and the molding characteristics are improved, and the resin becomes more superior on the aspects of economical and production efficiency such as the time reduction of the processing etc., furthermore the application range of the resin will be extended. The melting point of the epoxy resin greatly varies according to the content of the *cis*-isomer, so it is possible to control the melting point of the resin in the wide range from a room temperature to 150°C, and consequently to deal flexibly with the purposes and usages. The present invention provide the epoxy resin useful for adhesives, paints, and electric and electronic materials such as insulation materials and laminated plates etc.,and particularly useful to encapsulate electronic devices, and the process for producing it.

## Claims

1. A stilbene type epoxy resin represented by the general formula (1) consisting of 100 mol% of the *cis*-isomer, or 10 mol% or more of the *cis*-isomer and the rest of the *trans*-isomer 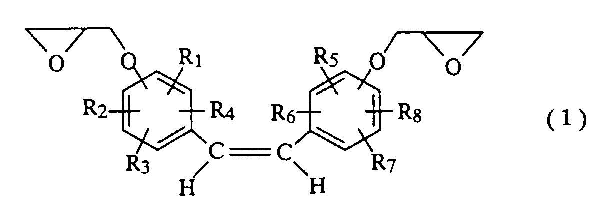 wherein R₁ to R₈ are independently an acyclic or cyclic alkyl group having 1 to 6 carbon atoms, hydrogen atom or a halogen atom.

2. A process for producing a stilbene type epoxy resin according to claim 1, characterized by the irradiation of a *trans*-stilbene type epoxy resin of the general formula (2) with ultra-violet light 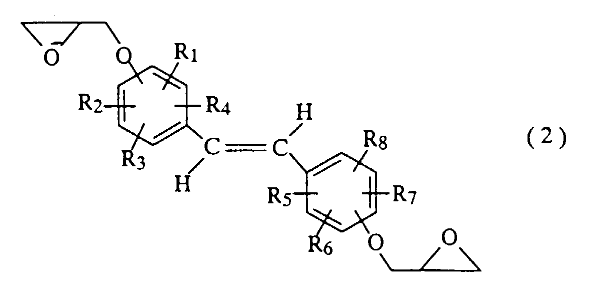 wherein R1 to R8 are independently an acyclic or cyclic alkyl group having 1 to 6 carbon atoms, hydrogen atom or a halogen atom.

3. A process for producing an epoxy resin according to claim 1, characterized by the irradiation of a *trans*-stilbene type phenol represented by the general formula (3) 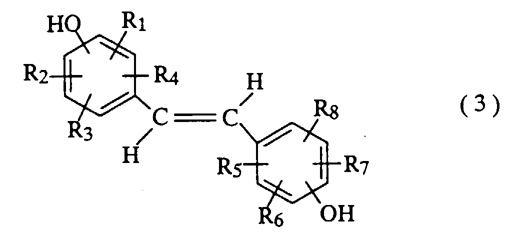 wherein R₁ to R₈ are independently an acyclic or cyclic alkyl group having 1 to 6 carbon atoms, hydrogen atom or a halogen atom), with ultra-violet light to obtain a stilbene type phenol represented by the general formula (4) consisting of 100 mol% of the *cis*-isomer, or 10 mol% or more of the *cis*-isomer and the rest of the *trans*-isomer 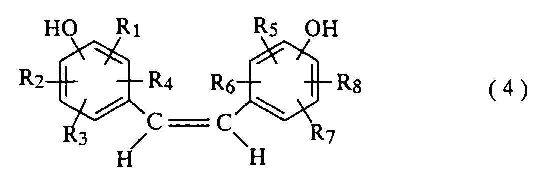 wherein R1 to R8 are as defined in the general formula (3), and by the reaction of the stilbene type phenol with epihalohydrin in the presence of basic substance.

## Patentansprüche

1. Epoxyharz vom Stilbentyp der allgemeinen Formel (1), bestehend aus 100 Mol-% des cis-Isomers oder aus 10 Mol-% oder mehr des cis-Isomers und zum Rest aus dem trans-Isomer 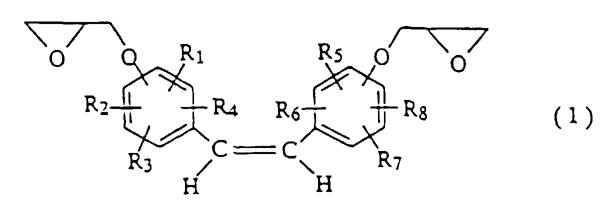 worin R₁ bis R₈ unabhängig voneinander für eine acyclische oder cyclische Alkylgruppe mit 1 bis 6 Kohlenstoffatom(en), ein Wasserstoffatom oder ein Halogenatom stehen.

2. Verfahren zur Herstellung eines Epoxyharzes vom Stilbentyp nach Anspruch 1, gekennzeichnet durch die Bestrahlung eines Epoxyharzes vom trans-Stilbentyp der allgemeinen Formel (2) 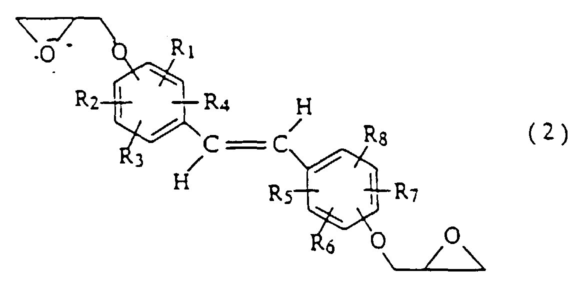 worin R₁ bis R₈ unabhängig voneinander für eine acyclische oder cyclische Alkylgruppe mit 1 bis 6 Kohlenstoffatom(en), ein Wasserstoffatom oder ein Halogenatom stehen, mit UV-Licht.

3. Verfahren zur Herstellung eines Epoxyharzes nach Anspruch 1, gekennzeichnet durch die Bestrahlung eines Phenols vom trans-Stilbentyp der allgemeinen Formel (3) 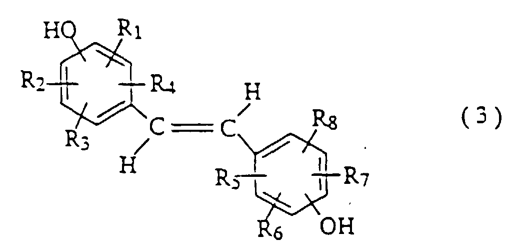 worin R₁ bis R₈ unabhängig voneinander für eine acyclische oder cyclische Alkylgruppe mit 1 bis 6 Kohlenstoffatom(en), ein Wasserstoffatom oder ein Halogenatom stehen, mit UV-Licht zur Gewinnung eines aus 100 Mol-% des cis-Isomers oder aus 10 Mol-% oder mehr des cis-Isomers und zum Rest aus dem trans-Isomer bestehenden Phenols vom Stilbentyp der allgemeinen Formel (4) 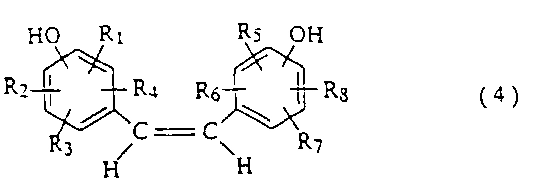 worin R₁ bis R₈ wie bei der allgemeinen Formel (3) definiert sind,
und die Umsetzung des Phenols vom Stilbentyp mit Epihalogenhydrin in Gegenwart einer basischen Substanz.

## Revendications

1. Résine époxy de type stilbène représentée par la formule générale (I) constituée de 100% en mol de l'isomère cis ou de 10% en mol ou plus de l'isomère cis et le reste étant l'isomère trans 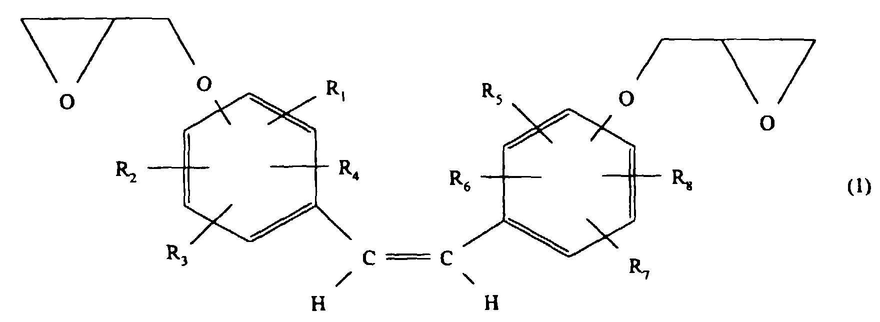 dans laquelle R₁ à R₈ sont indépendamment un groupe alkyle acyclique ou cyclique ayant de 1 à 6 atomes de carbone, un atome d'hydrogène ou un atome d'halogène.

2. Procédé de production d'une résine époxy de type stilbène selon la revendication 1, caractérisé par l'irradiation d'une résine époxy de type trans-stilbène de la formule générale (2) avec de la lumière ultraviolette 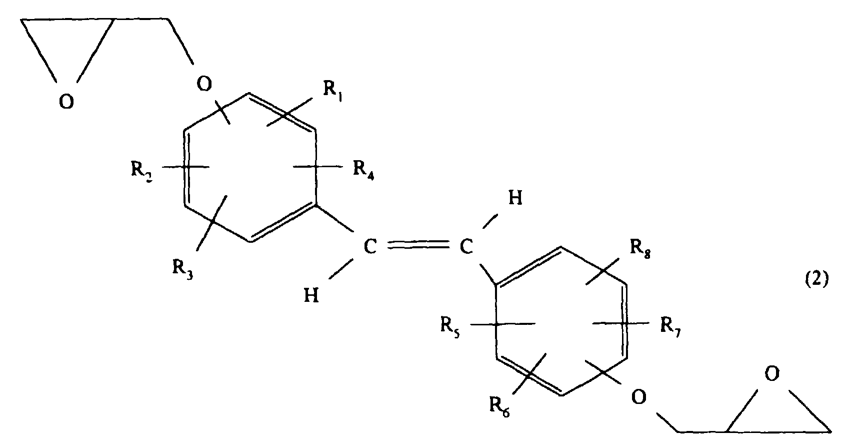 dans laquelle R₁ à R₈ sont indépendamment un groupe alkyle acyclique ou cyclique ayant de 1 à 6 atomes de carbone, un atome d'hydrogène ou un atome d'halogène.

3. Procédé de production d'une résine époxy selon la revendication 1, caractérisé par l'irradiation d'un phénol de type trans-stilbène représenté par la formule générale (3) 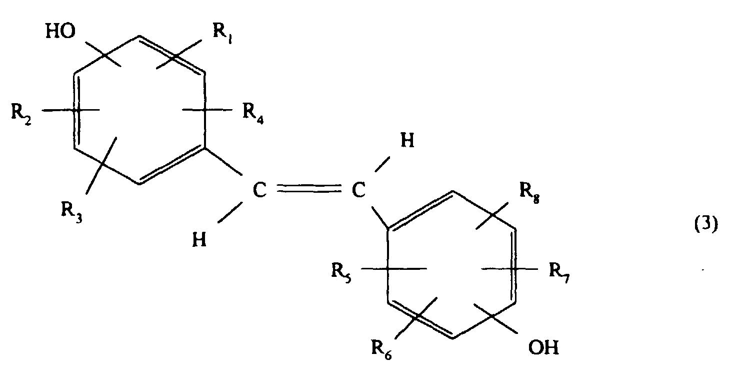 dans laquelle R₁ à R₈ sont indépendamment un groupe alkyle acyclique ou cyclique ayant de 1 à 6 atomes de carbone, un atome d'hydrogène ou un atome d'halogène avec de la lumière ultraviolette pour obtenir un phénol de type stilbène représenté par la formule générale (4) constitué de 100% en mol de l'isomère cis ou de 10% en mol ou plus de l'isomère cis et le reste étant l'isomère trans 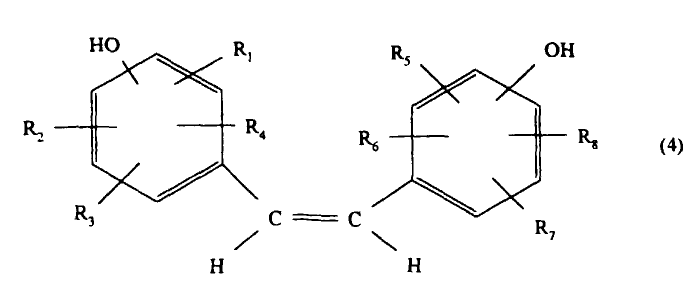 dans laquelle R₁ à R₈ sont comme définis dans la formule générale (3) et par la réaction du phénol de type stilbène avec de l'épihalohydrine en présence d'une substance basique.
